# EUROPEAN PATENT APPLICATION

(11) **EP 4 732 780 A1**
(43) Date of publication of application: **29.04.2026**
(21) Application number: 25208902.4
(22) Date of filing: 15.10.2025
(51) Int. Cl.: A61B 8/08, A61B 3/10, A61B 8/10, A61B 8/00

(54) **OPHTHALMIC ULTRASONIC DIAGNOSTIC DEVICE**

(30) Priority: 28.10.2024 JP 2024189216
(71) Applicant: Tomey Corporation, Nagoya-shi, Aichi 451-0051 (JP)
(72) Inventor: Owaki, Hirofumi, Nagoya-shi, 451-0051 (JP); Kawamura, Kenji, Nagoya-shi, 451-0051 (JP)
(74) Representative: RavensPAT Patentanwälte Partnerschaft mbB

(57) **Abstract**

An ophthalmic ultrasonic diagnostic device (1) according to an aspect of the present disclosure includes a probe (50) and a measurement instrument (20). The probe is configured to transmit an ultrasonic wave to the eye to be examined and receive a reflected wave of the ultrasonic wave from the eye to be examined. The measurement instrument is configured to output, as a measurement value, a length of a predetermined site of the eye to be examined, which is identified from the received signal, when the received signal by the probe satisfies a plurality of predetermined conditions. The measurement instrument is configured to cause a display device (41) to display a waveform of a received signal and an image (70) indicating a satisfaction level of a plurality of conditions, and cause the display device to switch a display state of the image according to the number of satisfied conditions among the plurality of conditions.

## Description

### CROSS-REFERENCE TO RELATED APPLICATIONS

The present application claims priority based on Japanese Patent Application No. 2024-189216 filed with the Japanese Patent Office on October 28, 2024, and the entire content of Japanese Patent Application No. 2024-189216 is incorporated herein by reference.

### BACKGROUND

The present disclosure relates to an ophthalmic ultrasonic diagnostic device.

As an ophthalmic device, a device that measures the length of an eye to be examined is known. Specifically, an ophthalmic ultrasonic diagnostic device that measures an anterior chamber depth (ACD), a lens thickness, and an ocular axial length of an eye to be examined is known (see Japanese Unexamined Patent Application Publication No. 2001-187022).

In an ophthalmic ultrasonic diagnostic device, an ultrasonic wave is transmitted from a probe toward an eye to be examined while the probe is in contact with the eye to be examined directly or through an ultrasonic medium, and a reflected wave thereof is received by the probe. The length of each site of the eye to be examined is measured based on the received signal by the probe.

When the eye to be examined is measured through the ultrasonic medium, the ultrasonic wave transmitted from the probe is reflected by the cornea, anterior lens surface, posterior lens surface, and retina of the eye to be examined. Therefore, in the received signal, peaks corresponding to reflected wave components from the cornea, the anterior lens surface, the posterior lens surface, and the retina appear as signal waveforms. The ACD, the lens thickness, and the ocular axial length described above are measured based on the elapsed time from the transmission of the ultrasonic wave to the reception of the reflected wave component corresponding to each peak and the propagation speed of the ultrasonic wave.

### SUMMARY

In the ophthalmic ultrasonic diagnostic device described above, the propagation path of the ultrasonic wave changes depending on the position and orientation (hereinafter, collectively referred to as "orientation") of the probe. Therefore, in order to accurately measure the eye to be examined, it is necessary to perform an operation of adjusting the probe to a correct orientation with respect to the eye to be examined. For example, for accurate measurement of the ocular axial length, it is necessary to arrange the probe so that ultrasonic waves propagate parallel to the eye axis. A slight inclination of the probe causes a measurement error of the ocular axial length.

In order to suppress such a measurement error caused by the orientation of the probe, the ophthalmic ultrasonic diagnostic device captures a measurement value based on the received signal only when the received signal by the probe satisfies a predetermined condition. Therefore, the operator of the probe cannot obtain the measurement value of the eye to be examined unless the operator performs an operation of adjusting the probe to the correct orientation with respect to the eye to be examined such that the received waveform satisfies the predetermined condition.

However, even when the operator adjusts the orientation of the probe for the measurement of the eye to be examined, the conventional device shows only a reaction to the extent that the measurement is performed or not performed. Therefore, in the conventional device, there is a possibility that the operator cannot correctly understand the situation and feels strong dissatisfaction with the fact that the measurement is not performed.

Therefore, according to an aspect of the present disclosure, it is desirable to provide an ophthalmic ultrasonic diagnostic device having excellent operability in measuring an eye to be examined.

According to an aspect of the present disclosure, an ophthalmic ultrasonic diagnostic device is provided. An ophthalmic ultrasonic diagnostic device includes a probe and a measurement instrument. The probe is configured to transmit an ultrasonic wave to the eye to be examined and receive a reflected wave of the ultrasonic wave from the eye to be examined.

The measurement instrument is configured to output, as a measurement value, a length of a predetermined site of the eye to be examined, which is identified from the received signal, when the received signal by the probe satisfies a plurality of predetermined conditions.

The measurement instrument is configured to cause the display device to display the waveform of the received signal, cause the display device to display the image indicating the satisfaction level of the plurality of conditions, and cause the display device to switch the display state of the image according to the number of satisfied conditions among the plurality of conditions.

According to the ophthalmic ultrasonic diagnostic device configured as described above, the operator of the probe can easily perform the work of obtaining the received signal satisfying the predetermined conditions by adjusting the orientation of the probe while viewing the image, compared with the case where there is no image. Therefore, according to an aspect of the present disclosure, it is possible to provide an ophthalmic ultrasonic diagnostic device having excellent operability in measuring an eye to be examined.

According to an aspect of the present disclosure, an image may include a plurality of image elements. Each of the plurality of image elements may correspond to one of the plurality of conditions. Each of the plurality of image elements may have a plurality of display modes having different display states.

In this case, the measurement instrument may be configured to cause the display device to display a plurality of image elements as images, and when one or more conditions among the plurality of conditions are satisfied, cause the display device to display one or more image elements corresponding to the satisfied one or more conditions among the plurality of image elements in a display mode different from display modes of the remaining image elements among the plurality of image elements. With this display, the measurement instrument can cause the display device to display the image so as to indicate the satisfaction level of the plurality of conditions.

According to the ophthalmic ultrasonic diagnostic device configured as described above, it is possible to display an image useful for easy orientation adjustment of the probe and acquisition of a received signal satisfying predetermined conditions to the user.

According to an aspect of the present disclosure, the plurality of display modes included in each of the plurality of image elements may include a plurality of display modes having different display colors. The measurement instrument may be configured to cause the display device to display an image so that one or more image elements are displayed in a color different from colors of the remaining image elements.

According to an aspect of the present disclosure, the measurement instrument may be configured to determine in a predetermined determination order whether the received signal satisfies each of the plurality of conditions. The measurement instrument may be configured to cause the display device to display an image in which a plurality of image elements corresponding to a plurality of conditions is disposed in the same order as the determination order.

According to the ophthalmic ultrasonic diagnostic device configured as described above, the image can be displayed in a display state according to the satisfaction level of a plurality of conditions, such as a level meter. Therefore, according to this ophthalmic ultrasonic diagnostic device, it is possible to display an image in a manner in which the user can easily understand the situation.

According to an aspect of the present disclosure, the measurement instrument may be configured to repeatedly determine whether the received signal satisfies a plurality of conditions and update the image. According to the ophthalmic ultrasonic diagnostic device configured as described above, the image can be updated so as to follow the reception state. Therefore, it is possible to display to the user an image useful for easy orientation adjustment of the probe and acquisition of the received signal satisfying the predetermined conditions.

According to an aspect of the present disclosure, the measurement instrument may be configured to output the measurement value by causing the display device to display the measurement value. According to the ophthalmic ultrasonic diagnostic device configured as described above, the operator of the probe can check the measurement value while checking the waveform and the image of the received signal through the same display device.

According to an aspect of the present disclosure, the measurement instrument may be configured to output, as a measurement value, one or more of an anterior chamber depth (ACD), a lens thickness, and an ocular axial length of the eye to be examined, which are identified from the received signal. According to such a configuration, it is possible to provide a device excellent in operability as an ophthalmic ultrasonic diagnostic device capable of measuring the ACD, the lens thickness, and/or the ocular axial length.

According to an aspect of the present disclosure, the plurality of conditions may include at least one of the following conditions:
- a condition related to the intensity of the reflected wave, the intensity being identified from the received signal,
- a condition related to a shape of the reflected wave, the shape being identified from a received signal, and
- a condition related to positions of reflected wave components, the positions being identified from received signal.

According to an aspect of the present disclosure, the plurality of conditions may differ according to the type of the eye to be examined. The image may vary depending on the type of eye to be examined. The measurement instrument may be configured to determine whether the received signal satisfies a plurality of conditions corresponding to the type of the eye to be examined, and cause the display device to display an image corresponding to the type of the eye to be examined.

The plurality of conditions may be changed according to the type of the eye to be examined. By causing the display device to display an image corresponding to the type of the eye to be examined, it is possible to provide an ophthalmic ultrasonic diagnostic device having excellent operability in measuring each type of the eye to be examined.

### BRIEF DESCRIPTION OF THE DRAWINGS

An example embodiment of the present disclosure will be described hereinafter with reference to the accompanying drawings, in which:
FIG. 1 is a block diagram illustrating a configuration of an ophthalmic ultrasonic diagnostic device;
FIG. 2 is a diagram illustrating a configuration of an exemplary measurement screen;
FIGs. 3A and 3B are diagrams illustrating waveforms of exemplary received signals;
FIG. 4 is a diagram illustrating measurement of an eye to be examined;
FIG. 5 is a flowchart illustrating an operation reception process performed by a processor;
FIG. 6 is a flowchart illustrating a measurement related process performed by a processor;
FIG. 7 is a diagram illustrating a configuration of a capturing condition definition table;
FIG. 8 is a diagram illustrating a change in a display state of an indicator image;
FIG. 9A is a diagram illustrating an indicator image for an overmature cataract eye or an IOL eye;
FIG. 9B is a diagram illustrating an indicator image for an aphakic eye or a shallow anterior chamber eye;
FIG. 10 is a flowchart illustrating an intensity condition determination process performed by a processor;
FIG. 11 is a flowchart illustrating a range condition determination process performed by a processor; and
FIG. 12 is a flowchart illustrating a waveform condition determination process performed by a processor.

### DETAILED DESCRIPTION OF EXEMPLARY EMBODIMENTS

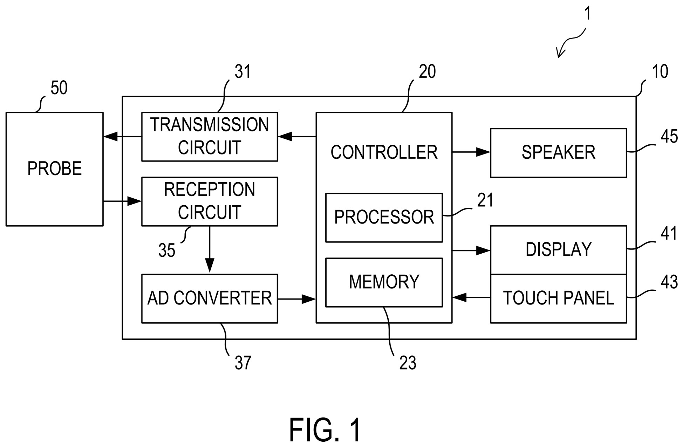

The ophthalmic ultrasonic diagnostic device 1 of the present embodiment illustrated in FIG. 1 is configured to measure the anterior chamber depth, the lens thickness, and/or the ocular axial length of the eye to be examined using the ultrasonic wave. Hereinafter, the anterior chamber depth is expressed as an ACD. The measurement of the ACD, the lens thickness, and/or the ocular axial length of the eye to be examined is referred to as measurement of the eye to be examined.

The ophthalmic ultrasonic diagnostic device 1 includes a device body 10 and an ultrasonic probe 50 connected to the device body 10 via a cable. Hereinafter, the ultrasonic probe 50 is simply referred to as a probe 50.

The device body 10 includes a controller 20, a transmission circuit 31, a reception circuit 35, an AD converter 37, a display 41, a touch panel 43, and a speaker 45. The transmission circuit 31 inputs a transmission signal to the probe 50 in accordance with a control signal from the controller 20. The transmission signal corresponds to a drive signal for the probe 50.

The probe 50 is configured to emit, that is, transmit, an ultrasonic wave of a corresponding waveform (for example, a pulse waveform) based on the transmission signal from the transmission circuit 31. The probe 50 includes a transducer (not illustrated), and converts a transmission signal as an electric signal into an ultrasonic wave to output the ultrasonic wave.

The probe 50 is configured to be able to receive a reflected wave of the emitted ultrasonic wave, converts the received ultrasonic wave into an electric signal, and outputs the electric signal as a received signal. The received signal is input to the reception circuit 35. The received signal indicates a waveform of an ultrasonic wave received by the probe 50, that is, a temporal change in the signal intensity, and includes reflected wave components of the emitted ultrasonic wave.

The reception circuit 35 includes an amplifier and a filter, amplifies the received signal, and reduces noise. The amplified and noise-reduced received signal is converted into a digital signal by the AD converter 37 and input to the controller 20.

The controller 20 is configured to control the probe 50 by inputting a control signal to the transmission circuit 31, and further measure the eye to be examined based on a received signal input through the AD converter 37. The controller 20 functions as a control device and a measurement instrument.

The controller 20 is further configured to provide a graphic user interface for the operator of the ophthalmic ultrasonic diagnostic device 1 through the display 41 and cause the speaker 45 to output a notification sound or the like according to the measurement status.

The controller 20 includes a processor 21 and a memory 23. The processor 21 performs processing related to measurement of the eye to be examined according to the computer program stored in the memory 23. The memory 23 may include a ROM, a RAM, and an NVRAM such as a flash memory. The memory 23 may include a storage, such as a solid state drive (SSD).

Although not illustrated, the controller 20 may further include a clock generation circuit and a counter circuit. The clock generation circuit is used to drive the probe 50. The counter circuit is used to measure an elapsed time from the time of ultrasonic wave emission.

The display 41 is an example of a display device, and is controlled by the controller 20 to display various types of information for the user of the ophthalmic ultrasonic diagnostic device 1. The user may be an operator of the probe 50. The display 41 is, for example, a liquid crystal display.

The touch panel 43 is configured integrally with the display 41, and is configured to receive a user's touch operation on a screen of the display 41 and input an operation signal thereof to the controller 20. The speaker 45 is configured to output a notification sound based on a control signal from the controller 20.

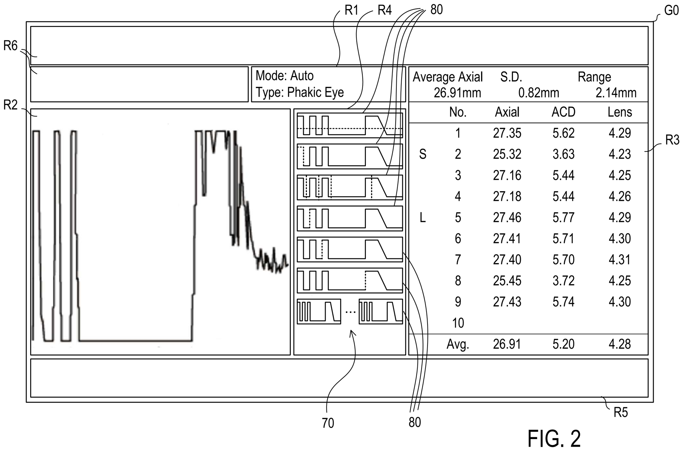

FIG. 2 illustrates a configuration of an exemplary measurement screen G0 displayed by the display 41. The measurement screen G0 includes a type display area R1, a waveform display area R2, a measurement value display area R3, an indicator image display area R4, an operation button display area R5, and other display areas R6.

The type display area R1 is an area for displaying the measurement mode and the type of the eye to be examined. According to the example illustrated in FIG. 2, the type display area R1 indicates that the measurement mode is the automatic measurement mode and the type of the eye to be examined is a phakic eye. An ophthalmic ultrasonic diagnostic device 1 of the present embodiment is configured to be able to measure a plurality of types of eyes to be examined including a phakic eye, an overmature cataract eye, an intra ocular lens (IOL) eye, an aphakic eye, and a shallow anterior chamber eye.

The waveform display area R2 is an area in which a waveform of a signal received by the probe 50 is displayed. According to the example illustrated in FIG. 2, in the waveform display area R2, the waveform of the received signal is displayed in a graph having a horizontal axis of time and a vertical axis of signal intensity.

The measurement value display area R3 is an area for displaying the measurement value of the eye to be examined. According to the example illustrated in FIG. 2, the measurement values of the respective times obtained by the plurality of times of measurement are listed together with the numbers indicating the measurement times. The measurement values include measurement values of the ocular axial length, the ACD, and the lens thickness.

The symbol "S" displayed left of the number is disposed at the head of the display row of the measurement value having the shortest ocular axial length among the measurement values displayed in the measurement value display area R3. The symbol "L" displayed left of the number is disposed at the head of the display row of the measurement value having the longest ocular axial length among the measurement values displayed in the measurement value display area R3.

The measurement value display area R3 includes an area in which the average, the standard deviation, and the fluctuation range of the ocular axial lengths obtained by the plurality of times of measurement is displayed in the upper part. The fluctuation range corresponds to a data range of the ocular axial length, and is a difference between a maximum value and a minimum value of measurement values of the ocular axial length obtained by a plurality of times of measurement. The measurement value display area R3 includes an area in which the averages of the ocular axial length, the ACD, and the lens thickness obtained by the plurality of times of measurements are displayed in the lower part.

The indicator image display area R4 is an area for displaying an indicator image 70 indicating the degree to which the received signal satisfies the measurement value capturing condition. The indicator image 70 may be understood to be an image that functions as an indicator. The capturing condition includes a set of a plurality of conditions. The degree to which the received signal satisfies the measurement value capturing condition, that is, the satisfaction level corresponds to the number or ratio of conditions satisfied by the received signal among the plurality of conditions constituting the capturing condition.

The indicator image 70 includes a plurality of image objects. The image object corresponds to an image element constituting the indicator image 70. Hereinafter, each of the plurality of image objects is referred to as a panel 80. Each of the plurality of panels 80 corresponds to one of the plurality of conditions constituting the capturing condition. Each of the plurality of panels 80 has a plurality of display modes having different display states. The plurality of display modes includes a first display mode and a second display mode having different display colors.

Each panel 80 is displayed in the first display mode when the received signal satisfies the corresponding condition, and is displayed in the second display mode when the received signal does not satisfy the corresponding condition.

That is, in the indicator image display area R4, one or more panels 80 corresponding to the conditions satisfied by the received signal are displayed in the first display mode, and the remaining panels 80 are displayed in the second display mode. For example, in the first display mode, the panel 80 is displayed in a bright color such as light green, and in the second display mode, the panel 80 is displayed in a dark color such as gray. Such display color switching effects turning on and off of the panel 80.

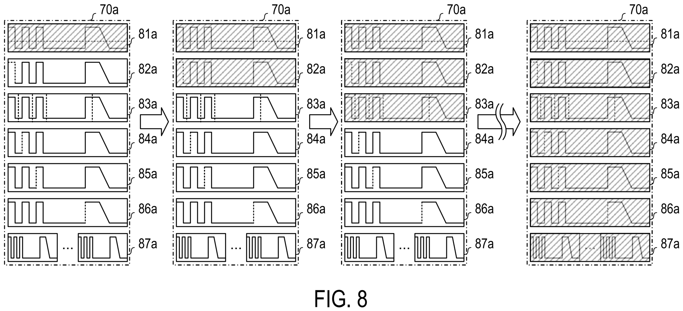

However, the display colors in the first display mode and the second display mode may be reversed. That is, in the first display mode, the panel 80 may be displayed in a dark color, and in the second display mode, the panel 80 may be displayed in a bright color. As described above, in the indicator image display area R4, the satisfaction level of the capturing condition is displayed through color switching of the plurality of panels 80, in other words, turning on and off of the plurality of panels 80 (see FIG. 8).

The operation button display area R5 is an area in which a plurality of operation buttons (not illustrated) that can be pressed by a user's touch operation are displayed as a graphical user interface. The other display area R6 includes an area for displaying information about the subject, an area for displaying the received signal intensity of the ultrasonic wave at the probe 50, and/or an area for displaying ultrasonic information such as frequency.

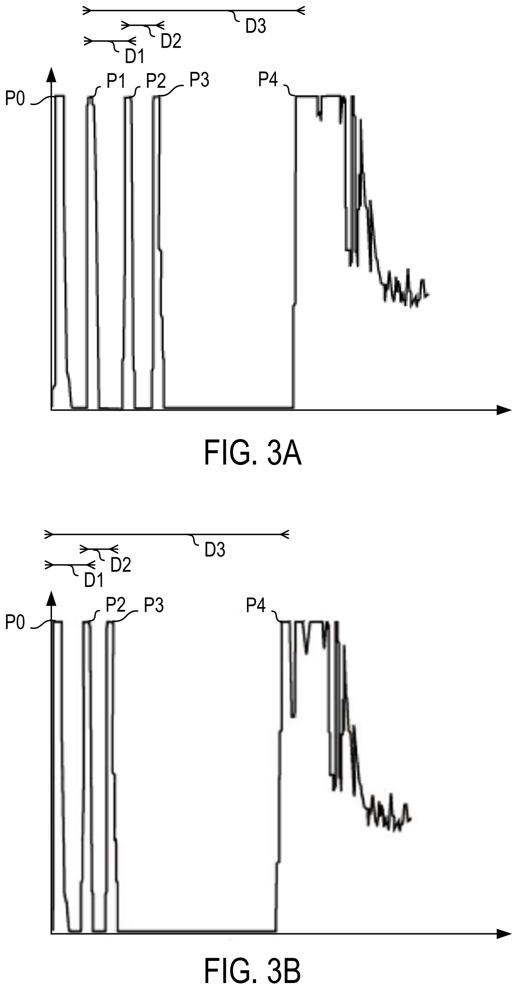

Next, a method of measuring the eye to be examined will be described with reference to FIGs. 3A and 3B together with the waveform of the received signal. When the eye to be examined is measured, the probe 50 is operated by the user of the ophthalmic ultrasonic diagnostic device 1, and the probe 50 is aligned with the eye to be examined.

The probe 50 is disposed to contact the corneal surface of the eye to be examined. Alternatively, the probe 50 is disposed to contact the corneal surface of the eye to be examined through the ultrasonic medium. The correct orientation of the probe 50 is an orientation in which an ultrasonic wave propagates coaxially with the eye axis of the eye to be examined. An alignment operation of the probe 50 is performed so that the probe 50 is in a correct orientation.

When the probe 50 is in the correct orientation, the ultrasonic wave from the probe 50 propagates from the cornea to the retina side of the eye to be examined so as to pass through the central axis of the eye to be examined. When the probe 50 contacts the corneal surface through the ultrasonic medium, the ultrasonic wave is reflected by the cornea, the anterior lens surface, the posterior lens surface, and the retina of the eye to be examined in this propagation process. The received signal includes a component reflected by the cornea, a component reflected by the anterior lens surface, a component reflected by the posterior lens surface, and a component reflected by the retina as reflection components of the ultrasonic wave transmitted from the probe 50, that is, reflected wave components.

A method of measuring the eye to be examined in a state where the probe 50 is brought into contact with the corneal surface through the ultrasonic medium is referred to as an immersion method. FIG. 3A illustrates a waveform of a received signal in a case where the eye to be examined is measured by the immersion method.

As another example, a method in which the probe 50 is brought into direct contact with the corneal surface to measure the eye to be examined is referred to as a contact method. In the case of the contact method, the ultrasonic wave is reflected at the anterior lens surface, the posterior lens surface, and the retina. In this case, the received signal includes a component reflected by the anterior lens surface, a component reflected by the posterior lens surface, and a component reflected by the retina as reflection components of the ultrasonic wave transmitted from the probe 50, that is, reflected wave components. FIG. 3B illustrates a waveform of a received signal in a case where the eye to be examined is measured by the contact method.

These reflected wave components indicate peaks of signal intensity in the received signal. Hereinafter, a reflected wave component appearing as a convex waveform having a peak in the received signal along with reception of the reflected wave is expressed as an echo. The received signal further includes an initial echo P0 due to a difference in acoustic impedance at the interface formed by contact between the probe 50 and the eye to be examined. The initial echo P0 can occur in both the immersion method and the contact method.

In the signal waveforms of the received signals illustrated in FIGs. 3A and 3B, a first echo P0 corresponds to an initial echo P0 caused by a difference in acoustic impedance, a second echo P1 corresponds to a reflected wave component from the cornea, a third echo P2 corresponds to a reflected wave component from the anterior lens surface, a fourth echo P3 corresponds to a reflected wave component from the posterior lens surface, and a fifth echo P4 corresponds to a reflection component from the retina.

Each of the echoes P1, P2, P3, and P4 appears temporally separated in the received signal according to the difference in the flight distance of the ultrasonic wave due to the difference in the reflection point. A time difference D1 between the first echo P1 and the second echo P2 corresponds to the ACD. A time difference D2 between the echo P2 and the echo P3 corresponds to the lens thickness. A time difference D3 between the echo P1 and the echo P4 corresponds to the ocular axial length. These time differences D1, D2, and D3 can be converted into distances by being multiplied by the propagation speed of the ultrasonic wave.

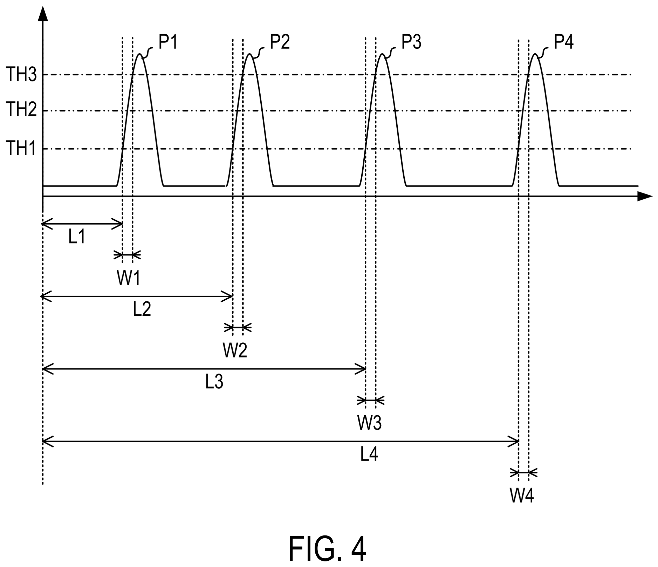

In the present embodiment, as illustrated in FIG. 4, the controller 20 measures the elapsed time from the time of emitting the ultrasonic wave to the time point at which the signal intensity of the received signal exceeds the predetermined threshold value TH1. As a result, elapsed times L1, L2, L3, and L4 from when the ultrasonic wave is emitted to when the reflected wave component corresponding to each of the echoes P1, P2, P3, and P4 is received are measured.

Based on the elapsed times L1, L2, L3, and L4 and the propagation speed of the ultrasonic wave, the distance corresponding to the time difference D1=L2-L1 is determined as the ACD, the distance corresponding to the time difference D2=L3-L2 is determined as the lens thickness, and the distance corresponding to the time difference D3=L4-L1 is determined as the ocular axial length.

When the probe 50 contacts the corneal surface, for example in the contact method, the time L1 converges to 0 because the transmission point of the ultrasonic wave from the probe 50 substantially coincides with the corneal surface. In this case, the controller 20 can determine the distances corresponding to the time difference D1=L2, the time difference D2=L3-L2, and the time difference D3=L4 as the ACD, the lens thickness, and the ocular axial length, respectively.

The controller 20 can detect each of the echoes P1, P2, P3, and P4 on condition that the signal intensity of the received signal sequentially exceeds a threshold value TH1, a threshold value TH2 larger than the threshold value TH1, and a threshold value TH3 larger than the threshold value TH2. However, even when the signal intensity does not exceed the threshold value TH3, the controller 20 may detect a peak of the corresponding signal intensity as an echo.

The controller 20 can further measure time widths W1, W2, W3, and W4 from when the signal intensity exceeds the threshold value TH1 to when the signal intensity reaches the threshold value TH3 in the echoes P1, P2, P3, and P4. Inverse numbers 1/W1, 1/W2, 1/W3, and 1/W4 of the time widths W1, W2, W3, and W4 correspond to rising slopes of the corresponding echoes P1, P2, P3, and P4, that is, sharpness of the rising. Hereinafter, the time widths W1, W2, W3, and W4 are referred to as rise times.

When the received signal satisfies a predetermined capturing condition based on the signal intensity of the received signal, the elapsed times L1, L2, L3, and L4, and the rise times W1, W2, W3, and W4, the controller 20 records measurement values (ACD, lens thickness, and ocular axial length) identified from the received signal, and controls the display 41 so that the measurement values are displayed in the measurement value display area R3.

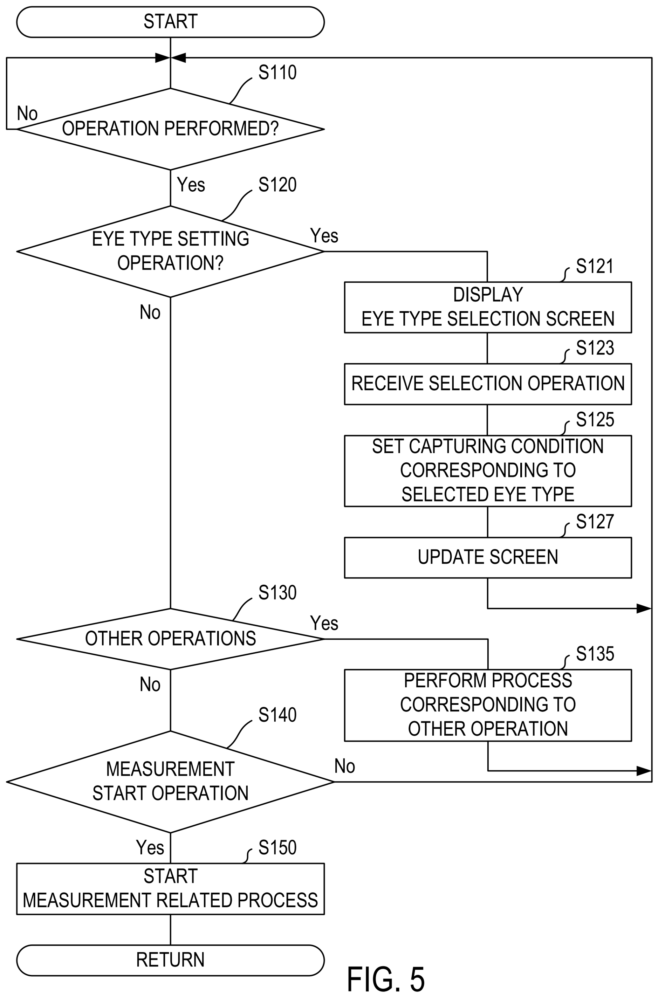

Next, details of the operation reception process performed by the processor 21 will be described with reference to FIG. 5. By repeatedly performing the operation reception process, the processor 21 receives operations on the operation button display area R5 of the measurement screen G0 from the user, and performs processing corresponding to each operation. Here, the user is an operator of the ophthalmic ultrasonic diagnostic device 1 and corresponds to the operator of the probe 50.

When the operation reception process is started, the processor 21 waits until an operation signal from the user is received through the touch panel 43 (S110). When receiving the operation signal, the processor 21 performs the process corresponding to the performed operation (S120 to S150).

When the performed operation is the setting operation of the type of the eye to be examined (Yes in S120), the processor 21 displays an eye type selection screen (S121). The operation button display area R5 is provided with operation buttons for receiving a setting operation of the type of the eye to be examined.

Although not illustrated, the eye type selection screen can be a screen on which the user can select, as a measurement target, one type from a group of types of the eyes that can be measured by the ophthalmic ultrasonic diagnostic device 1 through the touch panel 43. The eye type selection screen may be a screen on which one of a phakic eye, an overmature cataract eye, an IOL eye, an aphakic eye, and a shallow anterior chamber eye can be selected as a measurement target.

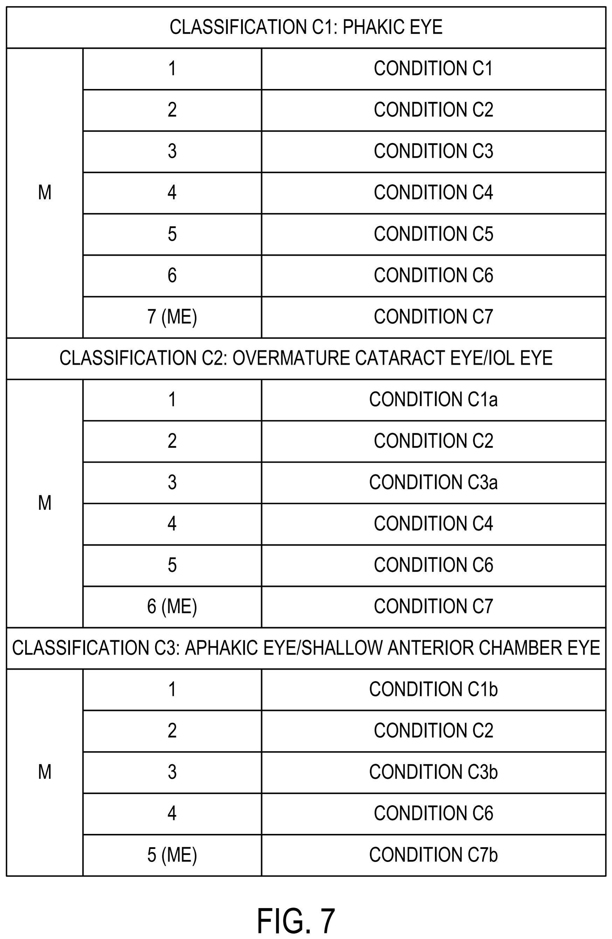

After displaying the eye type selection screen, the processor 21 receives an operation of selecting the type of the eye to be examined as the measurement target through the touch panel 43 (S123), and when the selection operation is performed, sets the measurement value capturing condition corresponding to the selected type of the eye to be examined (S125). The ophthalmic ultrasonic diagnostic device 1 can store, in the memory 23, a capturing condition definition table (see FIG. 7) that defines the capturing condition for each type of eye.

After setting the capturing condition, the processor 21 closes the eye type selection screen and displays the measurement screen G0 again. At this time, the measurement screen G0 is updated so that the selected type of the eye to be examined is displayed in the type display area R1 of the measurement screen G0 (S127). Thereafter, the processor 21 proceeds to S110 and waits until the next operation is performed.

When the performed operation is an operation other than the eye type setting operation and the measurement start operation (Yes in S130), the processor 21 performs processing corresponding to the performed operation (S135). Thereafter, the processor 21 proceeds to S110 and waits until the next operation is performed.

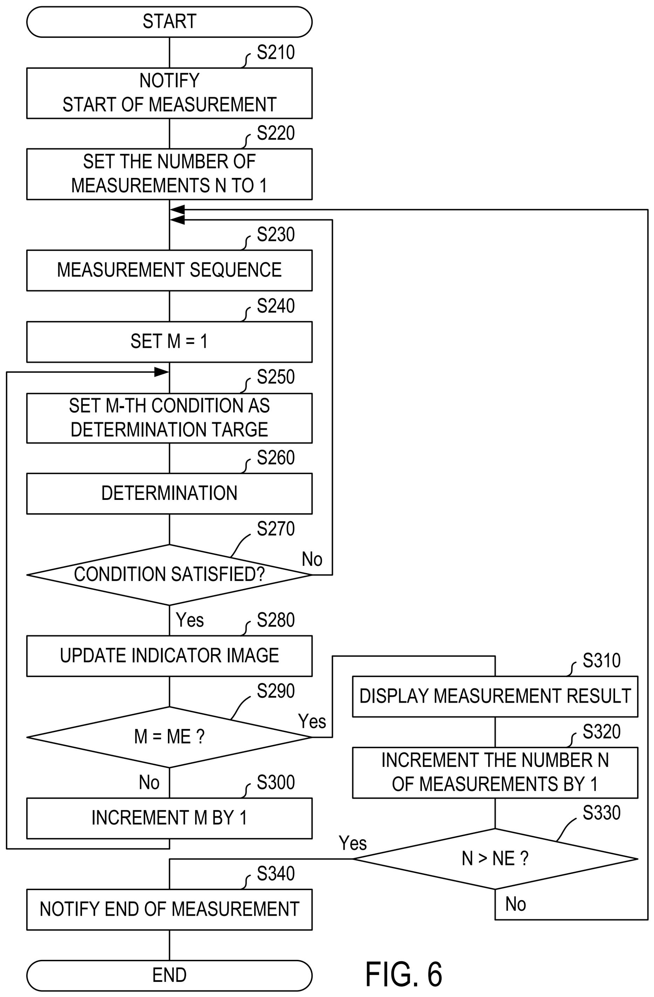

When the performed operation is the measurement start operation (Yes in S140), the processor 21 starts the measurement related process illustrated in FIG. 6 (S150). The operation button display area R5 is provided with an operation button for receiving a measurement start operation. After starting the measurement related process, the processor 21 ends the operation reception process.

Next, details of the measurement related process performed by the processor 21 will be described with reference to FIG. 6. When the measurement related process is started, the processor 21 notifies the user that the measurement is started (S210). The notification may be realized, for example, by causing the display 41 to display a message and/or by causing the speaker 45 to output a notification sound.

In subsequent S220, the processor 21 sets a variable N (hereinafter, expressed as the number of measurements N) for counting the number of measurements to a value 1. The number of measurements N corresponds to the number of the measurement displayed in the measurement value display area R3 (see FIG. 2).

In subsequent S230, the processor 21 starts a measurement sequence. In the measurement sequence, the processor 21 performs the transmission related process for causing the probe 50 to emit an ultrasonic wave and the reception related process for analyzing a received signal, from the probe 50, including reflected wave components of the ultrasonic wave.

The transmission related process includes a process of inputting the above-described control signal to the transmission circuit 31. The reception related process includes a process of measuring the signal intensity of the received signal and a process of measuring the elapsed times L1, L2, L3, and L4 and the rise times W1, W2, W3, and W4.

For example, the processor 21 may perform, as a measurement sequence, the transmission related process and the reception related process related to ultrasonic wave emission, reception, and analysis necessary for one measurement of the eye to be examined.

In subsequent S240, the processor 21 sets the variable M to a value 1. The variable M is a variable for controlling the condition under which the determination is to be made. In subsequent S250, the processor 21 sets, as a condition under which the determination is to be made, the M-th condition among the plurality of conditions constituting the predetermined capturing condition. Here, the sum of the plurality of conditions constituting the capturing condition is expressed as ME.

As described above, the plurality of conditions constituting the capturing condition is different for each type of the eye to be examined. When the type of the eye to be examined is a phakic eye, a plurality of conditions constituting the capturing condition is as follows. The following capturing conditions apply when the eye to be examined is measured in a contact method.
- Intensity condition C1: the signal intensity of each of echoes P2, P3, P4 of the anterior lens surface, the posterior lens surface, and the retina, respectively, exceeds a threshold value.
- Contact condition C2: the width (for example, half-value width) of the initial echo P0 is equal to or smaller than a reference width.
- Range condition C3: each of the measurement values of the ACD, the lens thickness, and the ocular axial length determined from the received signal is within a predetermined range.
- First waveform condition C4: the rise time W2 of the echo P2 of the anterior lens surface is equal to or less than a first reference value.
- Second waveform condition C5: the rise time W3 of the echo P3 of the posterior lens surface is equal to or less than a second reference value.
- Third waveform condition C6: the rise time W4 of the echo P4 of the retina is equal to or less than a third reference value.
- Dispersion condition C7: the dispersions of values of the ACD and the ocular axial length are within respective predetermined ranges.

The "dispersions of values of the ACD and the ocular axial length" described in the dispersion condition C7 correspond to dispersions of the ACD and the ocular axial length values based on a plurality of received signals obtained by transmitting and receiving ultrasonic waves a plurality of times including a past measurement sequence. The magnitude of the variation can be evaluated by a fluctuation range.

When the type of the eye to be examined is an overmature cataract eye or an IOL eye, a plurality of conditions constituting the capturing condition are as follows. The following capturing conditions apply when the eye to be examined is measured in a contact method.
- Intensity condition C1a: the signal intensity of each of the echoes P2 and P4 of the anterior lens surface and the retina, respectively, exceeds a threshold value.
- Contact condition C2: the width (for example, half-value width) of the initial echo P0 is equal to or smaller than a reference width.
- Range condition C3a: each of the measurement values of the ACD and the ocular axial length determined from the received signal is within a predetermined range.
- First waveform condition C4: the rise time W2 of the echo P2 of the anterior lens surface is equal to or less than a first reference value.
- Third waveform condition C6: the rise time W4 of the echo P4 of the retina is equal to or less than a third reference value.
- Dispersion condition C7: the dispersions of values of the ACD and the ocular axial length are within respective predetermined ranges.

The suffix "a" attached to the intensity condition C1a and the range condition C3a means that these conditions are variants of the intensity condition C1 and the range condition C3. The modification is based on a difference in the type of eye to be examined.

When the type of the eye to be examined is an aphakic eye or a shallow anterior chamber eye, a plurality of conditions constituting the capturing condition is as follows. The following capturing conditions apply when the eye to be examined is measured in a contact method.
- Intensity condition C1b: the signal intensity of the echo P4 of the retina exceeds a threshold value.
- Contact condition C2: the width (for example, half-value width) of the initial echo P0 is equal to or smaller than a reference width.
- Range condition C3b: each of the measurement value of the ocular axial length determined from the received signal is within a predetermined range.
- Third waveform condition C6: the rise time W4 of the echo P4 of the retina is equal to or less than a third reference value.
- Dispersion condition C7b: the dispersion of the value of the ocular axial length is within a predetermined range.

The suffix "b" attached to the intensity condition C1b, the range condition C3b, and the dispersion condition C7b means that these conditions are variants of the intensity condition C1, the range condition C3, and the dispersion condition C7. The modification is based on a difference in the type of eye to be examined.

As illustrated in FIG. 7, the capturing condition definition table that defines the capturing condition for each type of the eye to be examined defines the capturing condition and the determination order of the plurality of conditions constituting the capturing condition for each of the first classification, the second classification, and the third classification. Here, the phakic eye belongs to the first classification. The overmature cataract eye and the IOL eye belong to the second classification. The aphakic eye and the shallow anterior chamber eye belong to the third classification.

When the eye to be examined is in the first classification (phakic eye), the first condition is the intensity condition C1. The second condition is the contact condition C2. The third condition is the range condition C3. The fourth condition is the first waveform condition C4. The fifth condition is the second waveform condition C5. The sixth condition is the third waveform condition C6. The seventh condition is the dispersion condition C7. The ordinal numbers assigned to the first condition to the seventh condition correspond to the determination order.

When the eye to be examined is the second classification (overmature cataract eye or IOL eye), the first condition is the intensity condition C1a. The second condition is the contact condition C2. The third condition is the range condition C3a. The fourth condition is the first waveform condition C4. The fifth condition is the third waveform condition C6. The sixth condition is the dispersion condition C7.

When the eye to be examined is the third classification (aphakic eye or shallow anterior chamber eye), the first condition is the intensity condition C1b. The second condition is the contact condition C2. The third condition is the range condition C3b. The fourth condition is the third waveform condition C6. The fifth condition is the dispersion condition C7b.

In subsequent S260 (see FIG. 6), the processor 21 determines whether the received signal satisfies the M-th condition with respect to the M-th condition as a condition under which the determination is to be made. When determining that the M-th condition is not satisfied (No in S270), the processor 21 performs the process of S230. That is, the measurement sequence is performed again. Further, the processor 21 determines whether the capturing condition is satisfied again from the first condition (S240, S250, S260).

On the other hand, when determining that the received signal satisfies the M-th condition (Yes in S270), the processor 21 updates the indicator image 70 displayed in the indicator image display area R4 to a display state according to the number and the ratio of the conditions satisfied by the received signal to the plurality of conditions (S280).

That is, the processor 21 updates the indicator image 70 so that the panels 80 corresponding to the first to M-th conditions are displayed in the first display mode and the panels 80 corresponding to the M+ 1-th to ME-th conditions are displayed in the second display mode. As a result, the indicator image 70 is updated to a display state according to the satisfaction level of the plurality of conditions corresponding to the capturing condition (S280).

The indicator image 70 displayed on the measurement screen G0 includes the number of panels 80 corresponding to the type of the eye to be examined (see FIG. 2). The indicator image 70 includes the number of panels 80 corresponding to the total ME of the plurality of conditions in order to indicate the satisfaction level of the plurality of conditions constituting the capturing condition corresponding to the type of the eye to be examined. Each panel 80 is a waveform image illustrating a corresponding condition.

As illustrated in FIG. 8, an indicator image 70a displayed on the measurement screen G0 when the phakic eye is to be measured includes a first panel 81a corresponding to the first condition, a second panel 82a corresponding to the second condition, a third panel 83a corresponding to the third condition, a fourth panel 84a corresponding to the fourth condition, a fifth panel 85a corresponding to the fifth condition, a sixth panel 86a corresponding to the sixth condition, and a seventh panel 87a corresponding to the seventh condition. Here, the suffix "a" is attached to the reference signs of the indicator image 70a and the first to seventh panels 81a to 87a for the phakic eye. The indicator image 70a is a specific example of the indicator image 70 when the type of the eye to be examined is identified as the phakic eye. The first to seventh panels 81a to 87a correspond to the plurality of panels 80 included in the indicator image 70 described above.

FIG. 8 illustrates that, among the plurality of conditions, as the number of satisfied conditions increases, the number of panels 80 displayed in the first display mode, specifically, the number of lighting panels 80 increases, and conversely, the number of panels 80 displayed in the second display mode, specifically, the number of light-off panels 80 decreases.

An indicator image 70a illustrated first from the left in FIG. 8 indicates a state in which the first panel 81a corresponding to the first condition is turned on and the second to seventh panels 82a to 87a corresponding to the second condition to the seventh condition are turned off. An indicator image 70a illustrated second from the left in FIG. 8 illustrates a state in which the first panel 81a and the second panel 82a corresponding to the first condition and the second condition are turned on, and the third to seventh panels 83a to 87a corresponding to the third condition to the seventh condition are turned off.

A third indicator image 70a from the left in FIG. 8 illustrates a state in which the first to third panels 81a to 83a corresponding to the first to third conditions are turned on, and the fourth to seventh panels 84a to 87a corresponding to the fourth condition to the seventh condition are turned off. An indicator image 70a illustrated fourth from the left in FIG. 8 (in other words, first from the right) indicates a state in which the first to seventh panels 81a to 87a corresponding to the first to seventh conditions are turned on.

The indicator image 70 on the measurement screen G0 is switched to the indicator image 70 having the number of panels 80 corresponding to the type of the eye to be examined selected as the measurement target immediately before at the time of updating the measurement screen G0 in S127.

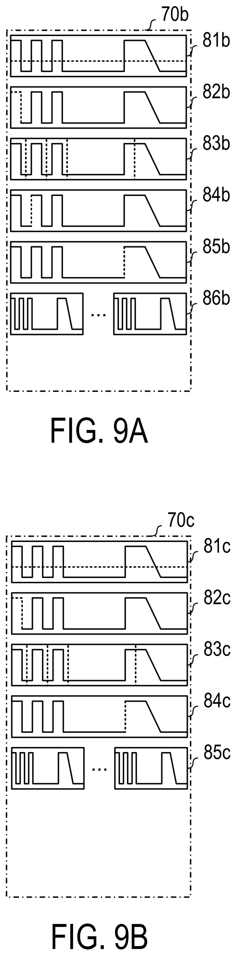

As illustrated in FIG. 9A, an indicator image 70b displayed on the measurement screen G0 when the overmature cataract eye or the IOL eye is to be measured includes a first panel 81b corresponding to the first condition, a second panel 82b corresponding to the second condition, a third panel 83b corresponding to the third condition, a fourth panel 84b corresponding to the fourth condition, a fifth panel 85b corresponding to the fifth condition, and a sixth panel 86b corresponding to the sixth condition. Here, the suffix "b" is attached to the reference signs of the indicator image 70b and the first to sixth panels 81b to 86b for the overmature cataract eye and the IOL eye. The indicator image 70b is a specific example of the indicator image 70 when the type of the eye to be examined is identified as the overmature cataract eye or the IOL eye. The first to sixth panels 81b to 86b correspond to the plurality of panels 80 included in the indicator image 70.

As illustrated in FIG. 9B, the indicator image 70 displayed on the measurement screen G0 when the aphakic eye or the shallow anterior chamber eye is to be measured includes a first panel 81c corresponding to the first condition, a second panel 82c corresponding to the second condition, a third panel 83c corresponding to the third condition, a fourth panel 84c corresponding to the fourth condition, and a fifth panel 85c corresponding to the fifth condition. Here, the suffix "c" is attached to the reference signs of the indicator image 70c and the first to fifth panels 81c to 85c for the aphakic eye and the shallow anterior chamber eye. The indicator image 70c is a specific example of the indicator image 70 when the type of the eye to be examined is identified as the aphakic eye or the shallow anterior chamber eye. The first to fifth panels 81c to 85c correspond to the plurality of panels 80 included in the indicator image 70.

When updating the indicator image 70 in S280, the processor 21 determines whether the variable M is the value ME corresponding to the sum of the plurality of conditions constituting the capturing condition (S290). This determination corresponds to determining whether the received signal satisfies all of the plurality of conditions constituting the capturing condition corresponding to the type of the eye to be examined.

When determining that the variable M is not the value ME in S290, the processor 21 updates the variable M to a value increased by 1 from the current value (S300), and proceeds to S250. In S250, the processor 21 sets the M-th condition corresponding to the updated variable M as a condition under which the determination is to be made.

Thereafter, the processor 21 determines whether the M-th condition is satisfied (S260), and when the M-th condition is satisfied (Yes in S270), the indicator image 70 is updated (S280). The update is performed so that the lighting panel 80 increases, for example. When the M-th condition is not satisfied, the processor 21 proceeds to S230, performs the reset to the variable M=1 (S240), and again determines whether the plurality of conditions is satisfied.

When determining that the variable M is the value ME in S290 (Yes in S290), the processor 21 displays the measurement values (ACD, lens thickness, and/or ocular axial length) identified from the received signal in the measurement sequence in S230 in the measurement value display area R3 (S310). Specifically, the processor 21 updates the measurement screen G0 displayed on the display 41 so that the measurement values are displayed on the N-th row of the measurement value display area R3 in association with the number N as the N-th measurement result (S310). As a result, the processor 21 outputs the measurement values through the display 41.

Thereafter, the processor 21 updates the number of measurements N to a value increased by 1 from the current value (S320). The processor 21 determines whether the number of measurements N exceeds the maximum number NE of displayable measurement values (S330). According to the example illustrated in FIG. 2, the maximum number NE=10.

When determining that the number of measurements N is equal to or smaller than the maximum number NE (No in S330), the processor 21 returns the process to S230 and starts the measurement of the eye to be examined in the N-th measurement (S230 to S300).

When determining that the number of measurements N exceeds the maximum number NE (Yes in S330), the processor 21 notifies the user of the end of the measurement through the output sound from the speaker 45 and the measurement screen G0 (S340), and then ends the measurement related process. The processor 21 can end the measurement related process while maintaining a state in which the measurement results up to the maximum number NE are displayed in the measurement value display area R3 of the measurement screen G0.

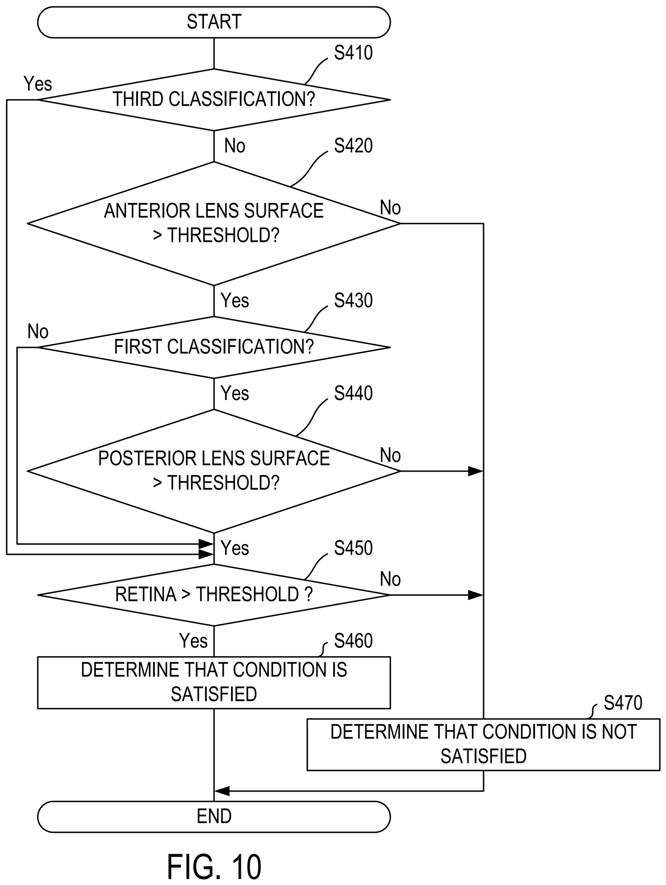

Although the details of the measurement related process have been described above, the method of determining the intensity conditions C1, C1a, and C1b will be specifically described as an example with reference to FIG. 10. When the M-th condition as a condition under which the determination is to be made is any of the intensity conditions C1, C1a, or C1b, the processor 21 can perform the intensity condition determination process illustrated in FIG. 10 in S260.

When the intensity condition determination process is started, the processor 21 determines whether the measurement target is the third classification (aphakic eye or shallow anterior chamber eye) (S410), and when the measurement target is the third classification (Yes in S410), the processor skips the process of S420, S430, and S440, and performs the process of S450.

On the other hand, when determining that the measurement target is a phakic eye, an overmature cataract eye, or an IOL eye other than the third classification (No in S410), the processor 21 determines whether the signal intensity of the echo P2 of the anterior lens surface exceeds the threshold value (S420).

When determining that the signal intensity of the echo P2 of the anterior lens surface is equal to or less than the threshold value (No in S420), the processor 21 determines that the received signal does not satisfy the intensity condition (S470). Determining that the received signal does not satisfy the intensity condition corresponds to determining that the received signal does not satisfy the M-th condition in S260.

When determining that the signal intensity of the echo P2 of the anterior lens surface exceeds the threshold value (Yes in S420), the processor 21 performs the process of S430. In S430, the processor 21 determines whether the measurement target is the first classification (phakic eye), and when the measurement target is not the first classification (No in S430), the processor skips the process of S440 and performs the process of S450. When the measurement target is the first classification (Yes in S430), the processor 21 determines whether the signal intensity of the echo P3 of the posterior lens surface exceeds the threshold value (S440).

When determining that the signal intensity of the echo P3 of the posterior lens surface is equal to or less than the threshold value (No in S440), the processor 21 determines that the received signal does not satisfy the intensity condition (S470). When determining that the signal intensity of the echo P3 of the posterior lens surface exceeds the threshold value (Yes in S440), the processor 21 performs the process of S450.

In S450, the processor 21 determines whether the signal intensity of the echo P4 of the retina exceeds the threshold value. When determining that the signal intensity of the echo P4 of the retina exceeds the threshold value (Yes in S450), the processor 21 determines that the received signal satisfies the intensity condition (S460). Determining that the received signal satisfies the intensity condition corresponds to determining that the received signal satisfies the M-th condition in S260.

On the other hand, when determining that the signal intensity of the echo P4 of the retina is equal to or less than the threshold value (No in S450), the processor 21 determines that the received signal does not satisfy the intensity condition (S470).

By performing such intensity condition determination process, the processor 21 can determine whether the received signal satisfies any of the intensity conditions C1, C1a, or C1b. The threshold value used for the determination in S420, S440, and S450 may be the threshold value TH1 illustrated in FIG. 4. The threshold value may be a different value in each of S420, S440, and S450.

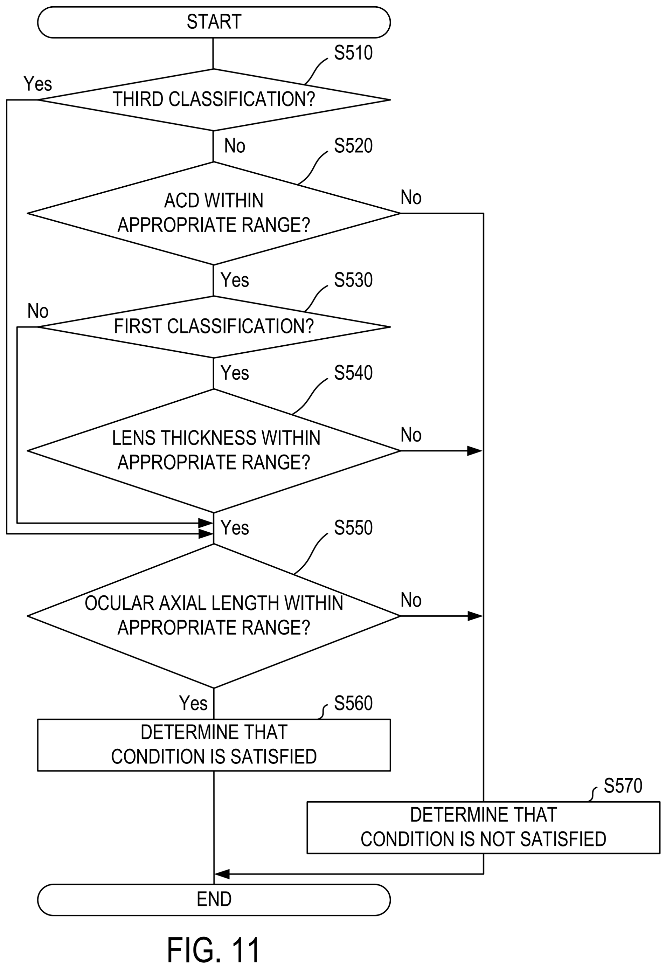

Next, a method of determining the range conditions C3, C3a, and C3b will be described with reference to FIG. 11. When the M-th condition as a condition under which the determination is to be made is any of the range conditions C3, C3a, or C3b, the processor 21 can perform the range condition determination process illustrated in FIG. 11 in S260. The range conditions C3, C3a, and C3b are conditions related to the positions of the reflected wave components included in the received signal.

When the range condition determination process is started, the processor 21 determines whether the measurement target is the third classification (S510), and when the measurement target is the third classification (Yes in S510), the processor skips the process of S520, S530, and S540 and performs the process of S550.

On the other hand, when the measurement target is a classification other than the third classification (No in S510), the processor 21 determines whether the ACD determined based on the difference between the elapsed times L1 and L2 is within the predetermined appropriate range (S520).

Here, when determining that the ACD is not within the appropriate range (No in S520), the processor 21 determines that the received signal does not satisfy the range condition (S570). Determining that the received signal does not satisfy the range condition corresponds to determining that the received signal does not satisfy the M-th condition in S260.

On the other hand, when determining that the ACD is within the appropriate range (Yes in S520), the processor 21 performs the process of S530. In S530, the processor 21 determines whether the measurement target is the first classification, and when the measurement target is not in the first classification (No in S530), the processor skips the process of S540 and performs the process of S550. When the measurement target is the first classification (Yes in S530), the processor 21 performs the process of S540.

In S540, the processor 21 determines whether the lens thickness determined based on the difference between the elapsed times L2 and L3 is within a predetermined appropriate range. When determining that the lens thickness is not within the predetermined appropriate range (No in S540), processor 21 determines that the received signal does not satisfy the range condition (S570). On the other hand, when determining that the lens thickness is within the appropriate range (Yes in S540), the processor 21 performs the process of S550.

In S550, the processor 21 determines whether the ocular axial length determined based on the difference between the elapsed times L1 and L4 is within a predetermined appropriate range. When determining that the ocular axial length is within the predetermined appropriate range (Yes in S550), the processor 21 determines that the received signal satisfies the range condition (S560). Determining that the received signal satisfies the range condition corresponds to determining that the received signal satisfies the M-th condition in S260.

On the other hand, when determining that the ocular axial length is not within the predetermined appropriate range, the processor 21 determines that the received signal does not satisfy the range condition (S570). By performing such range condition determination process, the processor 21 can determine whether the received signal satisfies any of the range conditions C3, C3a, or C3b.

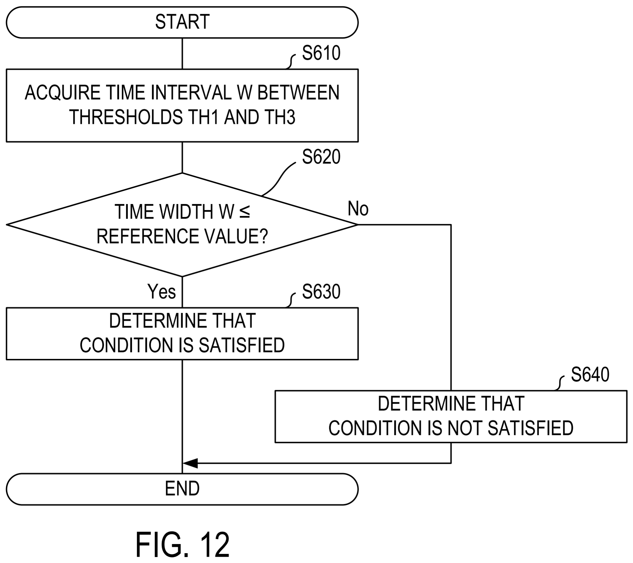

Next, a method of determining the first waveform condition C4, the second waveform condition C5, and the third waveform condition C6 will be described with reference to FIG. 12. Here, the first waveform condition C4, the second waveform condition C5, and the third waveform condition C6 are collectively expressed as waveform conditions C4, C5, and C6. The waveform conditions C4, C5, and C6 are conditions related to the shape of the reflected wave included in the received signal. When the M-th condition as a condition under which the determination is to be made is any of the waveform conditions C4, C5, or C6, the processor 21 can perform the waveform condition determination process illustrated in FIG. 12 in S260.

When the waveform condition determination process is started, the processor 21 acquires the time width W of the received signal in the process in which the signal intensity of the received signal rises from the threshold value TH1 to the threshold value TH3 (S610). When a condition under which the determination is to be made is the first waveform condition C4, the acquired time width W is the rise time W2 of the echo P2 of the anterior lens surface. When a condition under which the determination is to be made is the second waveform condition C5, the acquired time width W is the rise time W3 of the echo P3 of the posterior lens surface. When a condition under which the determination is to be made is the third waveform condition C6, the acquired time width W is the rise time W4 of the echo P4 of the retina.

Thereafter, the processor 21 determines whether the acquired time width W is equal to or less than the reference value (S620). The reference value is determined in consideration of the propagation speed of the ultrasonic wave. The reference value is an individual value corresponding to each of the waveform conditions C4, C5, and C6 as a condition under which the determination is to be made. That is, the reference value when a condition under which the determination is to be made is the first waveform condition C4 is the first reference value described above, the reference value when a condition under which the determination is to be made is the second waveform condition C5 is the second reference value, and the reference value when a condition under which the determination is to be made is the third waveform condition C6 is the third reference value.

When the time width W is equal to or less than the reference value (Yes in S620), the processor 21 determines that the received signal satisfies the waveform condition (S630). On the other hand, when the time width W exceeds the reference value (No in S620), the processor 21 determines that the received signal does not satisfy the waveform condition (S640).

By performing the waveform condition determination process in this manner, the processor 21 can determine whether the received signal satisfies any of the waveform conditions C4, C5, or C6. Determining that the received signal satisfies the waveform condition corresponds to determining that the received signal satisfies the M-th condition in S260. Determining that the received signal does not satisfy the waveform condition corresponds to determining that the received signal does not satisfy the M-th condition in S260.

According to the ophthalmic ultrasonic diagnostic device 1 of the present embodiment described above, the measurement value is captured and displayed in the measurement value display area R3 only when the capturing condition determined according to the type of the eye to be examined is satisfied. The capturing condition includes a plurality of conditions, and in the indicator image display area R4, the indicator image 70 is displayed in a display state according to the number and/or the ratio of the conditions satisfied by the received signal to the plurality of conditions constituting the capturing condition. The indicator image 70 has an image element (panel 80) corresponding to each condition.

The indicator image 70 effects turning on and off of the panels 80 like a level meter according to the number and/or the ratio of conditions satisfied by the received signal. Furthermore, each panel 80 represents a corresponding condition by a visual graphic symbol or an illustration instead of a character. The display state of the indicator image 70 changes substantially in real time following the change in the received signal.

Therefore, the ophthalmic ultrasonic diagnostic device 1 of the present embodiment can support the user's probe operation through the indicator image 70 until the user operating the probe 50 adjusts the orientation of the probe 50 so as to satisfy the measurement value capturing condition.

The user, that is, the operator of the probe 50 can easily adjust the probe 50 to the correct orientation by adjusting the orientation of the probe 50 while viewing the indicator image 70, compared with the case where there is no indicator image 70.

In the present embodiment, since the display of the panel 80 is switched by color as described above, it is easy for the user to visually understand the satisfaction level of the capturing condition. According to the present embodiment, since the arrangement order of the panels 80 constituting the indicator image 70 corresponds to the order of the condition determination by the processor 21, the display states of the panels 80 can be switched, in the manner of a level meter, according to the satisfaction level of the capturing condition.

Furthermore, in the present embodiment, the number and the arrangement of panels in the indicator image 70 displayed on the measurement screen G0 are switched in conjunction with the switching of the capturing condition according to the type of the eye to be examined. As described above, the indicator image 70 of the present embodiment is displayed in a form corresponding to the type of the eye to be examined, and is excellent in visibility of the satisfaction level of the capturing condition. Therefore, according to the present embodiment, it is possible to effectively support the orientation adjustment of the probe 50 in the direction in which the capturing condition is satisfied.

In addition, the indicator image 70 is displayed together with the waveform and the measurement values of the received signal on the measurement screen G0. Therefore, according to the present embodiment, it is possible to provide the ophthalmic ultrasonic diagnostic device 1 having excellent operability in measuring the eye to be examined.

### [Other Embodiments]

The present disclosure is not limited to the above embodiment and can be practiced in various forms. In the above embodiment, the number of panels in the indicator image 70 is switched according to the type of the eye to be examined.

However, the indicator image 70 having a uniform number of panels may be displayed on the measurement screen G0 regardless of the type of the eye to be examined. In this case, the indicator image 70 can be configured to include the number of panels 80 corresponding to the maximum number of conditions in accordance with the type of the eye to be examined having the largest number of conditions ME constituting the capturing condition. Panels 80 corresponding to conditions that do not need to be satisfied may be grayed out. This may indicate to the user that satisfaction of the conditions corresponding to the panels 80 is not necessary. Instead of graying out, the display state (the number of panels) of the indicator image 70 may be switched according to the type of the eye to be examined by a method of not displaying the unnecessary panels 80.

In addition, the ophthalmic ultrasonic diagnostic device 1 may be configured so that the capturing condition can be changed by the user. In this case, a button for changing the capturing condition may be prepared in the operation button display area R5 of the measurement screen G0. The processor 21 may be configured to receive operations of insertion and deletion of the panel 80 into and from the indicator image display area R4 when the button for changing a capturing condition is pressed. As a result, the processor 21 may receive a changing operation related to the arrangement of the panels 80 in the indicator image display area R4 and change the existing capturing condition to the capturing condition corresponding to the arrangement of the panels 80. Such a function of changing the capturing condition is convenient for a user who desires faster measurement than the accuracy of the measurement value.

In addition, the panel 80 for each condition included in the indicator image 70 may be configured to describe the corresponding condition by characters instead of graphical symbols or illustrations.

In the above embodiment, the capturing condition in the contact method is described in detail, but this capturing condition may be applied to the immersion method. When the type of the eye to be examined is a phakic eye, the capturing condition in the immersion method may be a capturing condition obtained by further adding the following interval condition to the above-described capturing condition in the contact method.
- Interval condition: the interval between the initial echo P0 and the echo P1 of the cornea is within a predetermined range.

The function of one constituent element in the above embodiment may be provided to be distributed to a plurality of constituent elements. The functions of the plurality of components may be integrated into one constituent element. Part of the configuration of the above embodiment may be omitted. Any embodiments included in the technical concept construed from the wording described in the claims are embodiments of the present disclosure.

## Claims

1. An ophthalmic ultrasonic diagnostic device (1) comprising:
a probe (50) configured to transmit an ultrasonic wave to an eye to be examined and to receive a reflected wave of the ultrasonic wave from the eye to be examined; and
a measurement instrument (20) configured to output, as a measurement value, a length of a predetermined site of the eye to be examined, the length being identified from a received signal, when the received signal by the probe (50) satisfies a plurality of predetermined conditions, wherein
the measurement instrument (20) causes a display device (41) to display a waveform of the received signal and an image (70) indicating a satisfaction level of the plurality of predetermined conditions, and causes the display device (41) to switch a display state of the image (70) according to the number of satisfied conditions among the plurality of predetermined conditions.

2. The ophthalmic ultrasonic diagnostic device (1) according to claim 1, wherein
the image (70) includes a plurality of image elements (80),
each of the plurality of image elements (80) corresponds to one of the plurality of predetermined conditions,
each of the plurality of image elements (80) has a plurality of display modes having different display states, and
the measurement instrument (20) causes the display device (41) to display the plurality of image elements (80) as the image (70), and when one or more conditions among the plurality of predetermined conditions are satisfied, causes the display device (41) to display one or more image elements corresponding to the one or more conditions among the plurality of image elements (80) in a display mode different from display modes of remaining image elements among the plurality of image elements (80).

3. The ophthalmic ultrasonic diagnostic device (1) according to claim 2, wherein
the plurality of display modes includes a plurality of display modes having different display colors, and
the measurement instrument (20) causes the display device (41) to display the image (70) so that the one or more image elements are displayed in a color different from colors of the remaining image elements.

4. The ophthalmic ultrasonic diagnostic device (1) according to claim 2 or 3, wherein
the measurement instrument (20) determines in a predetermined determination order whether the received signal satisfies each of the plurality of predetermined conditions, and causes the display device (41) to display the image (70) in which the plurality of image elements (80) corresponding to the plurality of predetermined conditions is disposed in a same order as the determination order.

5. The ophthalmic ultrasonic diagnostic device (1) according to any one of claims 1 to 4, wherein
the measurement instrument (20) repeatedly determines whether the received signal satisfies the plurality of predetermined conditions, and updates the image (70).

6. The ophthalmic ultrasonic diagnostic device (1) according to any one of claims 1 to 5, wherein
the measurement instrument (20) outputs the measurement value by causing the display device (41) to display the measurement value.

7. The ophthalmic ultrasonic diagnostic device (1) according to any one of claims 1 to 6, wherein
the measurement instrument (20) outputs, as the measurement value, one or more of an anterior chamber depth (ACD), a lens thickness, and an ocular axial length of the eye to be examined, which are identified from the received signal.

8. The ophthalmic ultrasonic diagnostic device (1) according to any one of claims 1 to 7, wherein
the plurality of predetermined conditions includes the following conditions:
(i) a condition related to an intensity of the reflected wave, the intensity being identified from the received signal;
(ii) a condition related to a shape of the reflected wave, the shape being identified from the received signal; and
(iii) a condition related to positions of reflected wave components, the positions being identified from the received signal.

9. The ophthalmic ultrasonic diagnostic device (1) according to any one of claims 1 to 8, wherein
the plurality of predetermined conditions differs according to a type of the eye to be examined,
the image (70) varies depending on the type of the eye to be examined, and
the measurement instrument (20) determines whether the received signal satisfies the plurality of predetermined conditions according to the type of the eye to be examined, and causes the display device (41) to display the image (70) according to the type of the eye to be examined.
